(19) Europäisches Patentamt
European Patent Office
Office européen des brevets

(11) Veröffentlichungsnummer: **0 287 866**
**A1**

(12) **EUROPÄISCHE PATENTANMELDUNG**

(21) Anmeldenummer: **88105148.6**

(22) Anmeldetag: **30.03.88**

(51) Int. Cl.⁴: **C07D 401/12 , C07D 401/14 , C07D 409/14 , C07D 413/14 , A61K 31/505**

(30) Priorität: **11.04.87 DE 3712371**

(43) Veröffentlichungstag der Anmeldung: **26.10.88 Patentblatt 88/43**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Franckowiak, Gerhard, Dr.**
**Henselweg 10**
**D-5600 Wuppertal 1(DE)**
Erfinder: **Rosentreter, Ulrich, Dr.**
**Kondorweg 23**
**D-5600 Wuppertal 1(DE)**
Erfinder: **Perzborn, Elisabeth, Dr.**
**Am Tescher Busch 13**
**D-5600 Wuppertal 11(DE)**
Erfinder: **Seuter, Friedel, Dr.**
**Mosspfad 16**
**D-5600 Wuppertal 1(DE)**
Erfinder: **Kayser, Michael, Dr.**
**Walter- Flex-Strasse 18**
**D-5090 Leverkusen(DE)**
Erfinder: **Thomas, Günther, Dr.**
**Via Campognallo 21/14**
**I-20020 Arese (Mi)(IT)**

(54) **Substituierte 1,4-Dihydropyridine, Verfahren zu ihrer Herstellung und ihre Verwendung.**

(57) Die Erfindung betrifft substituierte 1,4-Dihydropyridine der allgemeinen Formel I

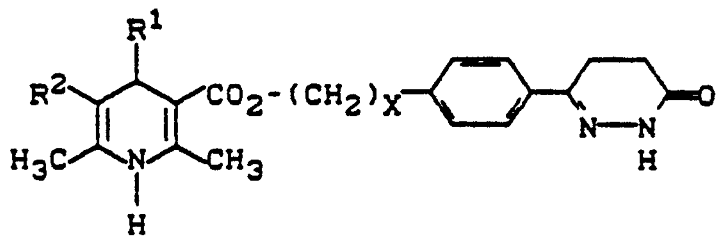

(I),

in welcher R¹, R² und X die in der Beschreibung angegebene Bedeutung haben, merhere Verfahren zu ihrer Herstellung und ihre Verwendung in Arzneimitteln, insbesondere zur Bekämpfung von Kreislauferkrankungen und Thrombosen.

EP 0 287 866 A1

## Substituierte 1,4-Dihydropyridine, Verfahren zur Herstellung und ihre Verwendung

Die Erfindung betrifft substituierte 1,4-Dihydropyridine, mehrere Verfahren zu ihrer Herstellung und ihre Verwendung in Arzneimitteln, insbesondere zur Bekämpfung von Kreislauferkrankungen und Thrombosen.

Die vorliegende Erfindung betrifft substituierte 1,4-Dihydropyridine der allgemeinen Formel (I)

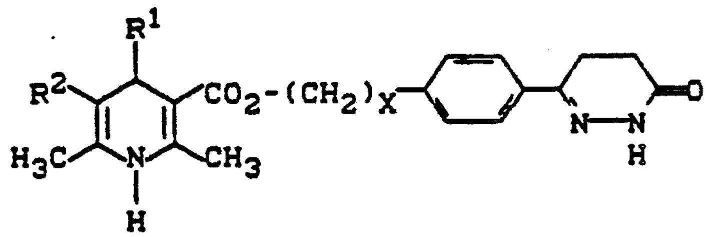

(I),

in welcher

$R^1$ - für $C_6$-$C_{14}$-Aryl steht, das bis zu vierfach gleich oder verschieden substituiert sein kann durch Halogen, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Trifluormethylthio oder durch gegebenenfalls durch Nitro, Cyano, Trifluormethyl, $C_1$-$C_4$-Alkyl oder $C_1$-$C_4$-Alkoxy substituiertes Benzyl, Benzyloxy oder Benzylthio, oder

-für einen gegebenenfalls durch Halogen, Phenyl oder $C_1$-$C_4$-Alkyl substituierten Heterocyclus aus der Reihe Pyrryl, Thienyl, Furyl, Pyrazolyl, Imidazolyl, Oxazolyl, Thiazolyl, Pyridyl, Pyridazinyl, Pyrimidyl, Chinolyl, Benzoxadiazolyl, Chromenyl oder Thiochromenyl steht, oder

-für geradkettiges, verzweigtes oder cyclisches Alkyl mit bis zu 6 Kohlenstoffatomen steht, das gegebenenfalls substituiert ist durch Pyridyl, Pyrimidyl, Halogen oder Nitro,

$R^2$ - für Cyano oder für eine Gruppe der Formel

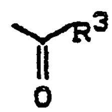

steht,

worin

$R^3$ - geradkettiges, verzweigtes oder cyclisches Alkyl mit bis zu 8 Kohlenstoffatomen bedeutet, oder

-eine Gruppe der Formel

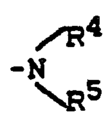

oder -$OR^6$ bedeutet,

worin

$R^4$, $R^5$ -gleich oder verschieden sind und

-für Wasserstoff, $C_1$-$C_6$-Alkyl, Phenyl oder Benzyl stehen,

und

$R^6$ -für Wasserstoff oder

-für geradkettiges, verzweigtes oder cyclisches Alkyl steht, das substituiert sein kann durch Halogen, Cyano, Hydroxy, $C_1$-$C_8$-Alkoxy, Pyridyl, Pyrimidyl, Thienyl, Furyl oder durch gegebenenfalls durch Halogen, Nitro, Cyano, Trifluormethyl, $C_1$-$C_4$-Alkyl, $C_1$-$C_4$-Alkoxy oder eine Gruppe der Formel

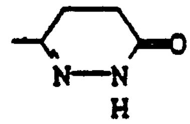

substituiertes Phenyl oder
durch Phenoxy, Phenylthio, Phenylsulfonyl oder
-durch eine Gruppe der Formel

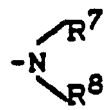 ,

worin
$R^7$, $R^8$ -gleich oder verschieden sind und
-für Wasserstoff, $C_1$-$C_6$-Alkyl, Phenyl, Benzoyl, Phenethyl, Acetyl, Benzoyl, Phenylsulfonyl oder $C_1$-$C_4$-Alkylsulfonyl stehen,
oder
$R^7$ und $R^8$ gemeinsam mit dem Stickstoffatom einen 5-bis 7-gliedrigen Ring bilden, der gegebenenfalls als weiteres Heteroatom ein Sauerstoff, Schwefel, NH, N-Phenyl oder N-$C_1$-$C_4$-Alkyl oder N-Benzyl enthalten kann,
und
X - für eine Zahl von 0 bis 6 steht
und deren physiologisch unbedenkliche Salze.

Bevorzugt sind Verbindungen der allgemeinen Formel (I),
in welcher
$R^1$ - für Phenyl oder Naphthyl steht, das bis zu dreifach gleich oder verschieden substituiert sein kann durch Fluor, Chlor, Brom, Nitro, Cyano, $C_1$-$C_4$-Alkyl, $C_1$-$C_4$-Alkoxy, $C_1$-$C_4$-Alkylthio, Trifluormethyl, Difluormethoxy, Trifluormethoxy, Trifluormethylthio, durch gegebenenfalls durch Nitro, Fluor, Chlor, Brom, Trifluormethyl, Methyl oder Methoxy substituiertes Benzyloxy oder Benzylthio, oder
-für einen gegebenenfalls durch Methyl, Fluor, Chlor, Brom oder Phenyl substituierten Heterocyclus aus der Reihe Thienyl, Furyl, Pyridyl, Pyrimidyl, Chinolyl, Benzoxadiazolyl oder Thiochromenyl steht, oder
-für geradkettiges oder verzweigtes, gegebenenfalls durch Fluor, Chlor, Brom oder Pyridyl substituiertes Alkyl mit bis zu 4 Kohlenstoffatomen steht,
$R^2$ - für Cyano oder für eine Gruppe der Formel

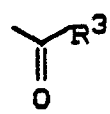

steht,
worin
$R^3$ - für geradkettiges, verzweigtes oder cyclisches Alkyl mit bis zu 6 Kohlenstoffatomen steht, oder
-für eine Gruppe der Formel

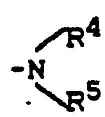

oder -$OR^6$ steht,
wobei
$R^4$, $R^5$ -gleich oder verschieden sind und

-für Wasserstoff, $C_1$-$C_4$-Alkyl oder Phenyl stehen,
und
$R^6$ -für Wasserstoff oder
-für geradkettiges oder verzweigtes Alkyl mit bis zu 8 Kohlenstoffatomen steht, das substituiert sein kann durch bis zu 7 Fluor, durch Chlor, Brom, Cyano, Hydroxy, $C_1$-$C_6$-Alkoxy, Pyridyl, Phenoxy oder durch gegebenenfalls durch Fluor, Chlor, Brom, Nitro, Cyano, Trifluormethyl, $C_1$-$C_2$-Alkyl, $C_1$-$C_2$-Alkoxy oder eine Gruppe der Formel

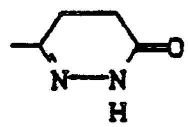

substituiertes Phenyl, oder
durch eine Gruppe der Formel

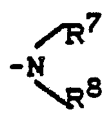 ,

wobei
$R^7$, $R^8$ -gleich oder verschieden sind und
- für Wasserstoff, $C_1$-$C_4$-Alkyl, Phenyl, Benzyl oder Acetyl stehen,
oder
$R^7$ und $R^8$ gemeinsam einen 5-bis 7-gliedrigen Ring bilden, der gegebenenfalls als weiteres Heteroatom Schwefel oder Sauerstoff enthält,
und
X - für eine Zahl von 0 bis 5 steht
und deren physiologisch unbedenkliche Salze.

Besonders bevorzugt sind Verbindungen der allgemeinen Formel (I),
in welcher
$R^1$ - für Phenyl steht, das bis zu zweifach gleich oder verschieden substituiert sein kann durch Fluor, Chlor, Nitro, Cyano, $C_1$-$C_4$-Alkyl, $C_1$-$C_4$-Alkoxy, Trifluormethyl, Benzyloxy oder Benzylthio, oder
-für Thienyl, Furyl, Pyridyl, Benzoxadiazolyl oder
-für einen Heterocyclus der Formel

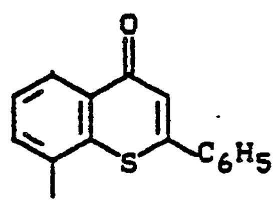

steht, oder
-für gegebenenfalls durch α-, β-oder γ-Pyridyl substituiertes Methyl oder Ethyl steht,
und
$R^2$ - für Cyano oder eine Gruppe der Formel

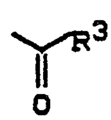

steht,

worin
$R^3$ - für geradkettiges oder verzweigtes Alkyl mit bis zu 4 Kohlenstoffatomen steht,
oder
-für einen Rest der Formel

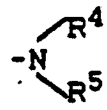

oder $-OR^6$ steht,
wobei
$R^4$, $R^5$ -gleich oder verschieden sind und
-für Wasserstoff, Methyl oder Ethyl stehen
und
$R^6$ -für Wasserstoff oder
-für geradkettiges oder verzweigtes Alkyl mit bis zu 6 Kohlenstoffatomen steht, das durch bis zu 3 Fluor, Chlor, Brom, Cyano, Hydroxy, $C_1$-$C_4$-Alkoxy, α-, β-oder γ-Pyridyl oder durch eine Gruppe der Formel

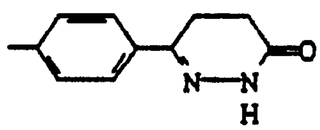

oder

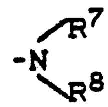

substituiert sein kann,
worin
$R^7$, $R^8$ -gleich oder verschieden sind und
-für Wasserstoff, Methyl, Ethyl oder Benzyl stehen,
oder
$R^7$ und $R^8$ gemeinsam einen Pyrrolidino-oder Piperidinoring bilden,
und
X - für eine Zahl 1 bis 4 steht
und deren physiologisch unbedenkliche Salze.

Physiologisch unbedenkliche Salze sind Salze der erfindungsgemäßen Verbindungen mit anorganischen oder organischen Säuren. Hierzu gehören bevorzugt anorganische Säuren wie Halogenwasserstoffsäuren, bevorzugt HCl oder HBr, Schwefelsäure, Phosphorsäure, oder organische Carbonsäuren oder Sulfonsäuren wie beispielsweise Essigsäure, Maleinsäure, Fumarsäure, Äpfelsäure, Citronensäure, Weinsäure, Milchsäure, Benzoesäure, Methansulfonsäure, Ethansulfonsäure, Benzolsulfonsäure, Naphthalindisulfonsäure oder Toluolsulfonsäure.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen der allgemeinen Formel (I) werden hergestellt,
indem man

[A] Aldehyde der allgemeinen Formel (II)

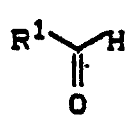

(II)

in welcher
$R^1$ die angegebene Bedeutung hat,

mit Ketonen der allgemeinen Formel (III),

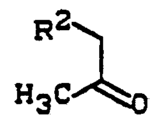

(III)

in welcher
$R^2$ die angegebene Bedeutung hat,
und Enaminen der allgemeinen Formel (IV)

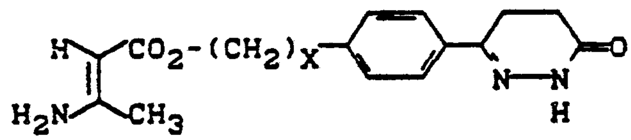

(IV)

in welcher
X die angegebene Bedeutung hat,
in Gegenwart inerter Lösemittel umsetzt,
oder indem man

[B] Aldehyde der allgemeinen Formel (II), mit Ketonen der allgemeinen Formel (V),

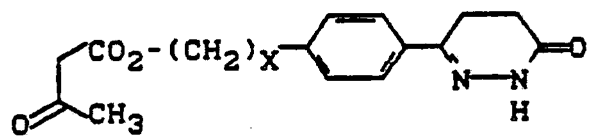

(V)

in welcher
X die angegebene Bedeutung hat,
und Enaminen der allgemeinen Formel (VI)

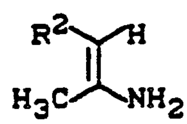

(VI),

in welcher
$R^2$ die angegebene Bedeutung hat,
in Gegenwart inerter Lösemittel umsetzt,
oder indem man

[C] Ketone der allgemeinen Formel (III) mit Ammoniak und Ylidenverbindungen der allgemeinen Formel (VII)

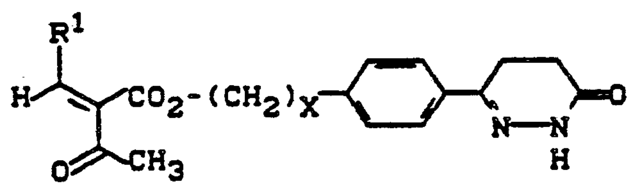

(VII)

in welcher
$R^1$ und X die angegebene Bedeutung haben,
in Gegenwart inerter Lösemittel umsetzt,
oder indem man

[D] Ketone der allgemeinen Formel (V), mit Ammoniak und Ylidenverbindungen der allgemeinen Formel (VIII)

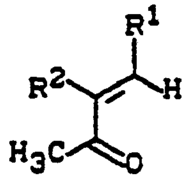

(VIII)

in welcher
$R^1$ und $R^2$ die angegebene Bedeutung haben,
in Gegenwart inerter Lösemittel umsetzt,
oder indem man

[E] Enamine der allgemeinen Formel (VI) mit Ylidenverbindungen der allgemeinen Formel (VII) in Gegenwart inerter Lösemittel umsetzt,
oder indem man

[F] Enamine der allgemeinen Formel (IV) mit Ylidenverbindungen der allgemeinen Formel (VII) in Gegenwart inerter Lösemittel umsetzt.

Je nach Art der eingesetzten Ausgangsverbindungen lassen sich die Verfahrensvarianten A - F durch folgende Schemata verdeutlichen:

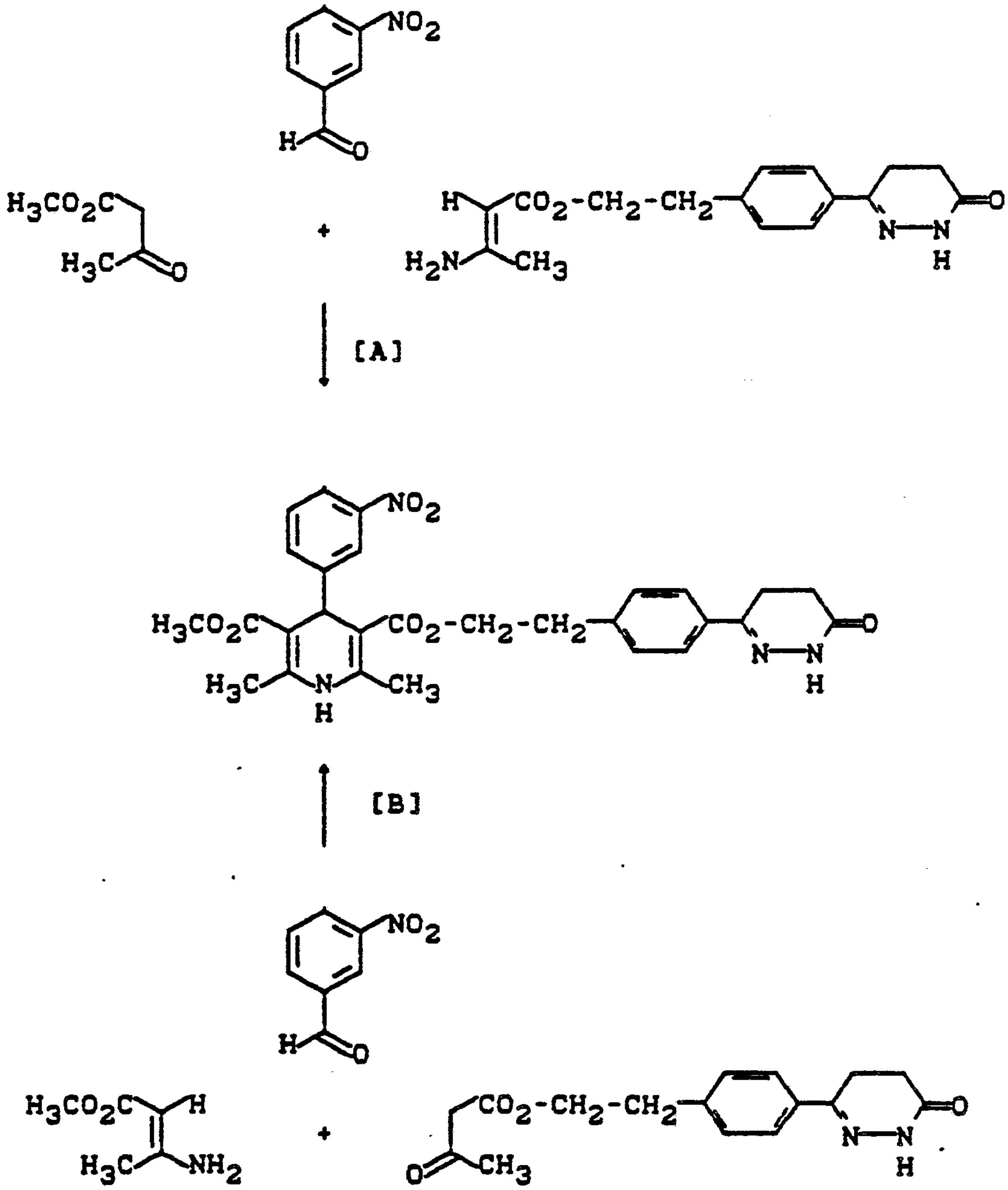
[A]
[B]

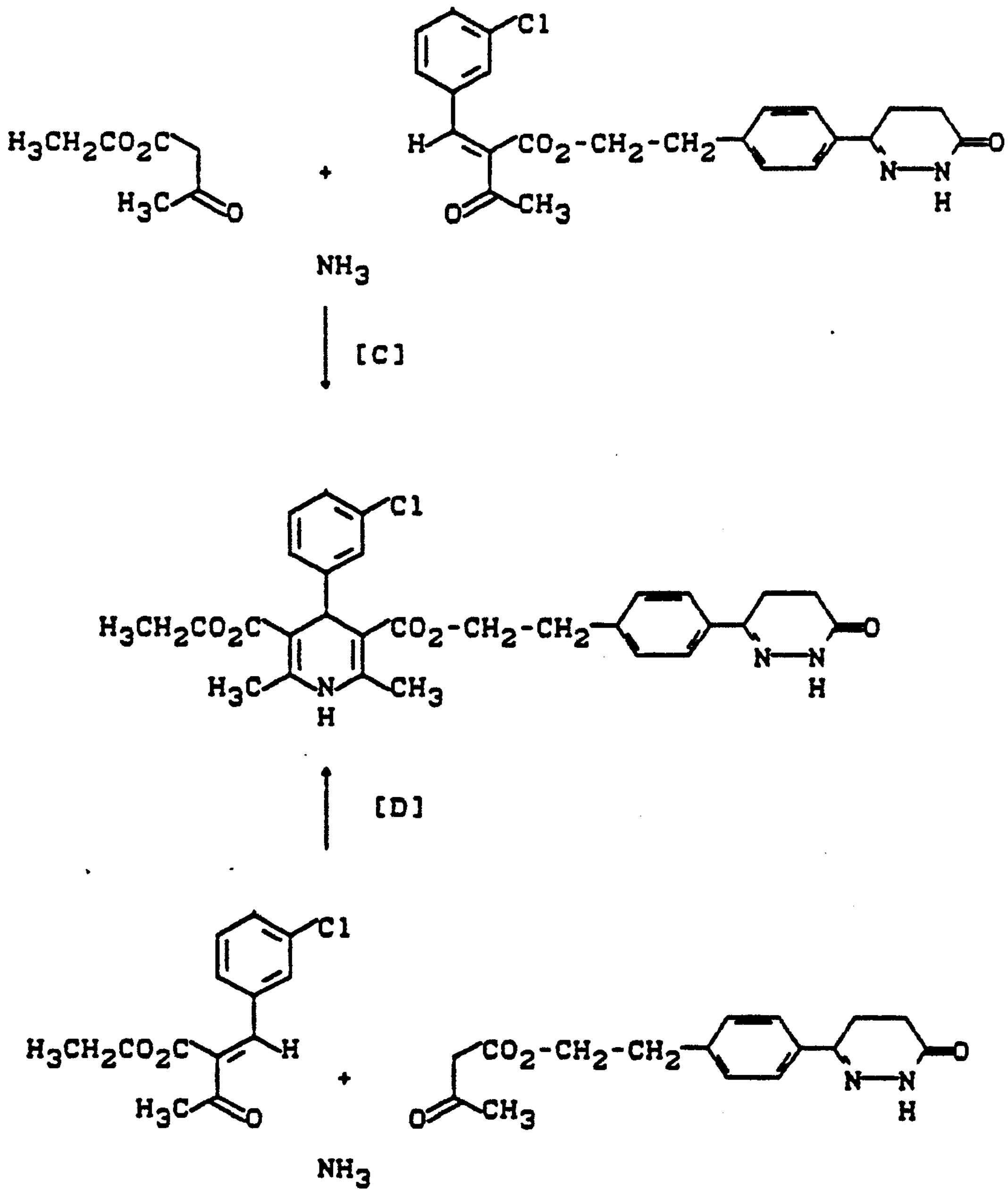
$NH_3$
[C]
[D]
$NH_3$

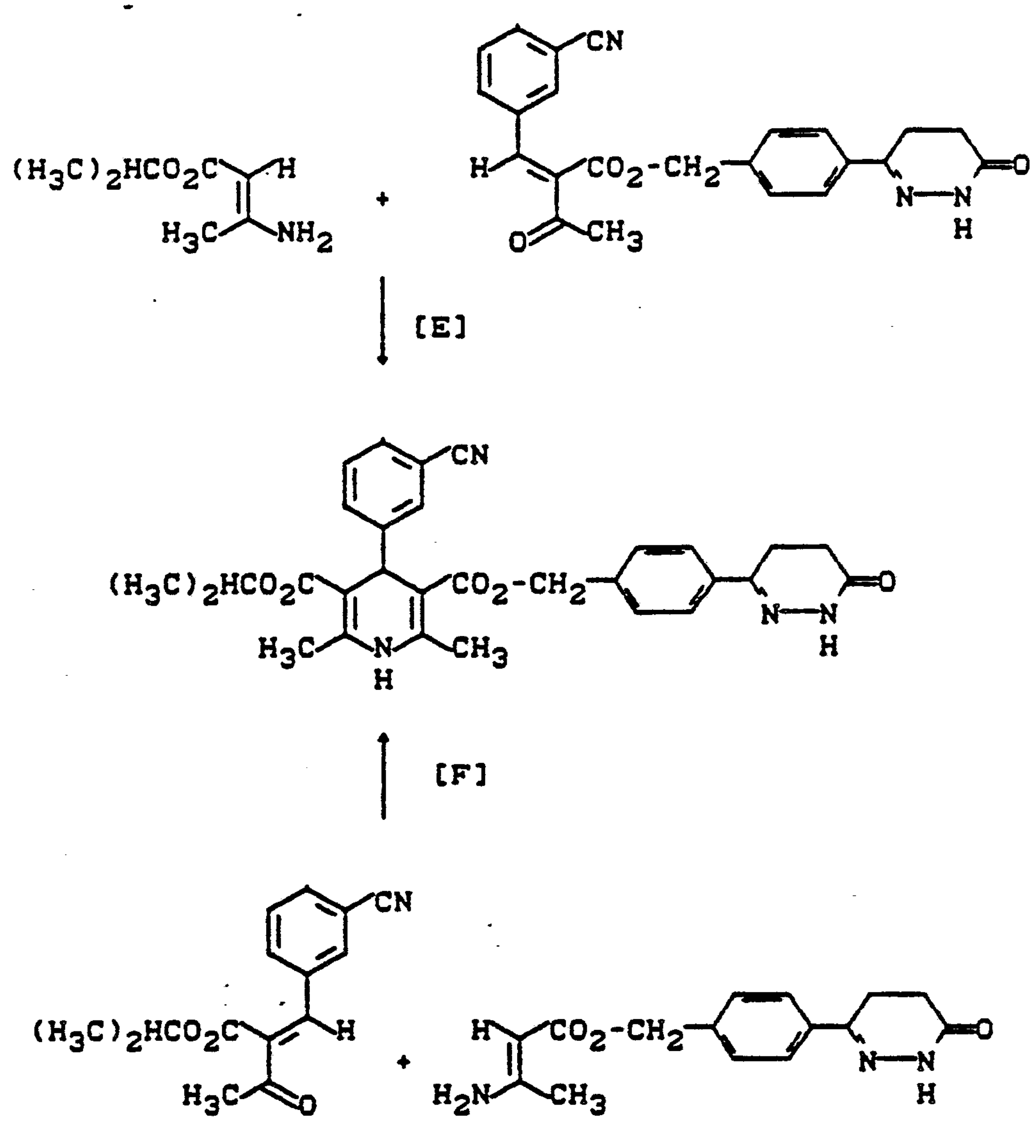


Die als Ausgangsstoffe eingesetzten Aldehyde der allgemeinen Formel (II) sind bekannt oder können nach bekannten Methoden hergestellt werden [E. Mosettig, Organic Reactions III, 218, (1954); E.P. Papadopoulos, A. Jarrar, C.H. Isidorides, J. Org. Chem. 31, 615 (1966); A.J. Mancho, D.S. Brownfain, D. Swern, J. Org. Chem. 44, 4148 (1979)].

Die als Ausgangsstoffe eingesetzten Ketone der allgemeinen Formeln (III) und (IV) sind bekannt oder können nach bekannten Methoden hergestellt werden [D. Borrmann, in Houben-Weyls "Methoden der organischen Chemie" VII/4, 230 (1968)].

Die als Ausgangsstoffe eingesetzten Enamine der allgemeinen Formeln (V) und (VI) sind bekannt oder können nach bekannten Methoden hergestellt werden [S.A. Glickman, A.C. Cope, J. Am. Chem. Soc. 67, 1017 (1945)].

Die als Ausgangsstoffe eingesetzten Ylidenverbindungen der allgemeinen Formeln (VII) und (VIII) sind bekannt oder können nach bekannten Methoden hergestellt werden [G. Jones, Organic Reactions XV, 204 (1967)].

Als Lösemittel für Verfahren A - F können Wasser und alle inerten organischen Lösemittel verwendet werden, die sich unter den Reaktionsbedinungen nicht verändern. Hierzu gehören bevorzugt Alkohole wie Methanol, Ethanol, Propanol, Isopropanol, Ether wie Diethylether, Dioxan oder Tetrahydrofuran, Dimethylformamid, Dimethylsulfoxid, Acetonitril, Eisessig, Essigester, Hexamethylphosphorsäuretriamid, oder Kohlenwasserstoffe wie Benzol, Toluol oder Xylol. Ebenso können Gemische der genannten Lösemittel eingesetzt werden.

Die Reaktionstemperaturen für alle Verfahren können in einem größeren Bereich variiert werden. Im allgemeinen arbeitet man in einem Bereich von +10°C bis +200°C, bevorzugt von +20°C bis 150°C, insbesondere bei der Siedetemperatur des verwendeten Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung des erfindungsgemäßen Verfahrens ist das Verhältnis der an der Reaktion beteiligten Stoffe beliebig. Im allgemeinen arbeitet man mit molaren Mengen der Reaktanden. Bei Verfahren C und D hat es sich als zweckmäßig erwiesen, Ammoniak im bis zu 20fachen, bevorzugt bis zu 10fachen Überschuß einzusetzen.

Die erfindungsgemäßen Verbindungen zeigen ein nicht vorhersehbares wertvolles pharmakologisches Wirkspektrum. Die erfindungsgemäßen Stoffe der Formel (I) wirken als Hemmer/Stimulatoren von enzymatischen Reaktionen im Rahmen des Arachidonsäurestoffwechsels. Derartige Substanzen sind zur Verhütung und Behandlung von Erkrankungen der Atemwege wie Emphysem, Schocklunge, pulmonaler Hypertonie, Ödem, Thrombose und Thromboembolie, Ischämien (periphere, coronare, cerebrale Durchblutungsstörungen), Herz-und Hirninfarkte, Herzrhythmusstörungen, Angina pectoris, Hypertonie sowie Arteriosklerose geeignet. Die erfindungsgemäßen Stoffe sind bevorzugt Hemmer der Thromboxansynthese, stimulieren gleichzeitig die Prostacyclinsynthese und wirken thrombozytenaggregationshemmend.

Die Wirkungen der erfindungsgemäßen Verbindungen werden durch folgende Experimente nachgewiesen:

## I. $^3H$-Arachidonsäuremetabolismus

Der Arachidonsäuremetabolismus in Human-Thrombozyten wurde mit Hilfe von Tritium-markierter Arachidonsäure untersucht. Die Thrombozyten metabolisieren die Arachidonsäure über den Cyclooxygenaseweg zu $TXA_2$ und HHT und über den Lipoxygenaseweg zu 12-HETE, die dünnschichtchromatographisch getrennt werden können [vgl. Bailey, J. M. et al., Prostaglandins 13, 479-492, (1977)]. Inhibitoren der einzelnen enzymatischen Reaktionen verändern das chromatographische Verteilungsmuster in charakteristischer Weise.

Gewaschene Human-Thrombozyten von gesunden Spendern, die 14 Tage kein Medikament eingenommen hatten, wurden mit Prüfsubstanz 2 min bei 37°C inkubiert und anschließend mit $^3H$-Arachidonsäure weitere 10 min bei 37°C inkubiert. Die Suspension wurde angesäuert und mit Essigester extrahiert. Der Essigester wurde unter Stickstoffatmosphäre abgedampft und der Rückstand in Methanol/Trichlormethan (1:1) aufgenommen und auf DC-Plastikfolien aufgetragen. Die Trennung erfolgte mit einem Fließmittelgemisch Trichlormethan/Methanol/Eisessig/Wasser (80:8:1:0.8). Die Verteilung der Radioaktivität wurde mittels eines Radioscanners gemessen (Tabelle 1).

## II: Prostacyclinstimulation

Außerdem stimulieren die erfindungsgemäß zu verwendenden 1,4-Dihydropyridine die Synthese von $PGI_2$. $PGI_2$ wirkt im Gegensatz zu dem vasokonstriktorischen und thrombozytenaggregationsauslösenden Thromboxan gefäßdilatierend zu thrombozytenaggregationshemmend.

### Stimulation im Vollblut

Im Vollblut läßt sich durch Kollagen die Bildung von $PGI_2$ induzieren. Die in Thrombozyten gebildeten Endoperoxide werden wahrscheinlich durch Leukozyten-Lipoxygenase in $PGI_2$ umgewandelt. Das stabile Endprodukt der $PGI_2$-Umwandlung, 6-Keto-$PGF_{1\alpha}$ wird radioimmunologisch bestimmt (Tabelle 2).

## III. Thrombozytenaggregation

Thrombozyten und deren Adhäsion - sowie Aggregationsfähigkeit - sind, besonders im arteriellen Schenkel des Gefäßsystems, ein wesentlicher pathogenetischer Faktor bei der Entstehung von Thrombosen.

Zur Bestimmung der thrombozytenaggregationshemmenden Wirkung wurde Blut von gesunden Probanden beiderlei Ge schlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP) (Jürgen/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Born, G.V.R., J. Physiol. (London), 162, 67, 1962) im Aggregometer bei 37°C bestimmt (Therapeutische Berichte 47, 80 - 86, 1975). Hierzu wurde die vorinkubierte Probe mit 0,1 ml Kollagen, einem aggregationsauslösenden Agens, versetzt. Die Veränderung der optischen Dichte in der Probe des PRP wurde während einer Zeitdauer von 4 Minuten aufgezeichnet und der Ausschlag nach 4 Minuten bestimmt. Hierzu wird die prozentuale Hemmung gegenüber der Kontrolle errechnet.
Als Grenzkonzentration wird der Bereich der minimal effektiven Konzentrationen angegeben (Tabelle 3).

**Tabelle 1**

Hemmung der $TXA_2$-Synthese

| Bsp.-Nr. | Grenzkonzentration für Hemmung MEC [µg/ml] |
|---|---|
| 11 | 0,1 |
| 16 | 0,1 |
| 18 | 0,1 |
| 19 | 0,1 |
| 21 | 1 - 0,3 |
| 22 | < 0,1 |

**Tabelle 2**

Prostacyclinstimulation

| Bsp.-Nr. | Stimulation der $PGI_2$-Synthese MEC [µg/ml] |
|---|---|
| 11 | 1 - 0,1 |
| 21 | < 3 |
| 22 | < 1 |

Tabelle 3

Hemmung der Thrombozytenaggregation

| Bsp.-Nr. | Grenzkonzentration für Hemmung MEC [µg/ml] |
|---|---|
| 11 | 1 - 0,3 |
| 12 | 1 - 0,3 |
| 24 | 1 - 0,3 |
| 15 | 3 - 1 |
| 16 | 1 - 0,3 |
| 17 | 1 - 0,3 |
| 18 | 1 - 0,3 |
| 19 | 1 - 0,3 |
| 20 | 1 - 0,3 |
| 10 | > 10 |
| 21 | > 10 |
| 22 | 10 - 3 |
| 7 | 3 - 1 |
| 23 | 10 - 3 |
| 24 | 3 - 1 |
| 9 | 3 - 1 |

Darüberhinaus beeinflussen die erfindungsgemäßen Verbindungen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz, eingesetzt werden. Darüberhinaus können sie zur Behandlung von Herzrhythmusstörungen, zur Senkung des Blutzuckers, zur Abschwellung von Schleimhäuten und zur Beeinflussung des Salz-und Flüssigkeitshaushaltes verwendet werden.

Die Herz-und Gefäßwirkungen wurden am isoliert perfundierten Herzen des Meerschweinchens gefunden.

Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energieliefemdes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem

Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965) 529 -541). Der Perfusionsdruck wird mittels eines Druckaufnehmers, der vor dem Herzen mit dem Perfusionssystem in Verbindung steht, registiert. Unter diesen Bedingungen zeigt eine Senkung des Perfusionsdrucks eine Koronardilatation, eine Zu-bzw. Abnahme der linksventrikulären Kontraktionsamplitude einen Anstieg bzw. eine Senkung der Herzkontraktilität an. Die erfindungsgemäßen Verbindungen werden in geeigneten Verdünnungen in das Perfusionsmedium kurz vor dem isolierten Herzen infundiert.

Die folgenden Werte zeigen beispielhaft den Effekt der erfindungsgemäßen Verbindungen am isoliert perfundierten Meerschweinchenherzen, ausgedrückt als prozentuale Differenz gegenüber dem gleich 100% gesetzten Ausgangswert.

| Bsp.- Nr. | Konzentration (g/ml) | %-Änderung der Kontraktionskraft | %-Änderung des Perfusionsdruckes |
|---|---|---|---|
| 7 | $10^{-6}$ | -60 | 0 |
| 11 | $10^{-6}$ | -23 | -36 |
| 13 | $10^{-6}$ | -78 | 0 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Waser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

4-Oxo-4-[4-(tetrahydropyran-2-yl-oxymethyl)phenyl]-buttersäure

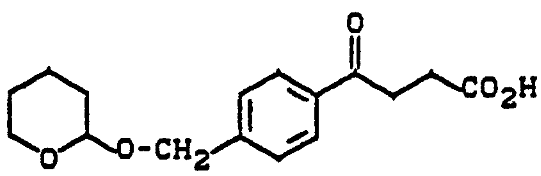

Unter Schutzgas werden 12,75 g (0,53 mol) Magnesium-Späne in 200 ml Tetrahydrofuran mit wenig Iod aktiviert, dann läßt man 135 g p-Brombenzyl-tetrahydropyranylether langsam zutropfen. Die Reaktion verläuft stark exotherm. Zur vollständigen Umsetzung läßt man 2 h unter Erwärmen nachrühren. Die Lösung wird auf -80°C gekühlt und 53 g Bernsteinsäureanhydrid, suspendiert in 200 ml Tetrahydrofuran, werden portionsweise zugegeben. Es wird 2 h nachgerührt, dann mit 4 l Wasser hydrolysiert und mit 2 N Natronlauge auf pH 8 eingestellt. Es wird mit Methylenchlorid gewaschen. Die wäßrige Phase wird mit Citronensäure auf pH 4,5 eingestellt. Das Produkt wird mit Methylenchlorid extrahiert, getrocknet und durch Zugabe von Petrolether ausgefällt.
Schmp.: 97°C
Ausbeute: 59 g (40,4% der Theorie)

### Beispiel 2

[4-(6-Oxo-1,4,5,6-tetrahydropyridazin-3-yl)benzyl]tetrahydropyran-2-yl-ether

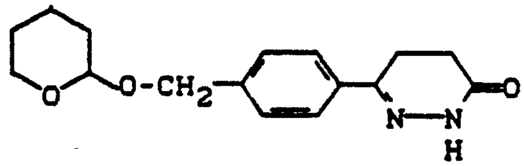

50 g 4-Oxo-4-[4-(tetrahydropyran-2-yl-oxymethyl)-phenyl]-buttersäure (Beispiel 1) werden in 240 ml Wasser suspendiert und mit 10 ml Hydrazinhydrat 3 h bei 90°C verrührt. Nach dem Abkühlen saugt man die ausgefallenen Kristalle ab. Die Wasserphase wird mit Methylenchlorid extrahiert, dieses wird getrocknet und eingedampft. Der Rückstand wird mit Ether verrieben.
Ausbeute: 42 g (84% der Theorie)

### Beispiel 3

4-(6-Oxo-1,4,5,6-tetrahydropyridazin-3-yl)benzylalkohol

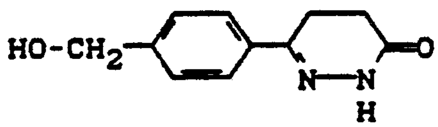

48 g [4-(6-Oxo-1,4,5,6-tetrahydropyridazin-3-yl)benzyl]tetrahydropyran-2-yl-ether (Beispiel 2) werden in 600 ml Tetrahydrofuran gelöst und mit je 100 ml Eisessig, 1 n Salzsäure und Wasser versetzt. Man läßt 24 h bei Raumtemperatur stehen, destilliert das Tetrahydrofuran ab und extrahiert den Rückstand mit Methylenchlorid. Dabei fällt das Produkt bereits teilweise aus und wird abgesaugt. Die Methylenchloridphase wird aufgearbeitet. Beide Kristallisate werden vereinigt.
Schmp.: 188°C
Ausbeute: 26,3 g (74% der Theorie)

Beispiel 4

Acetessigsäure-[4-(6-oxo-1,4,5,6-tetrahydropyridazin-3-yl)benzyl]ester

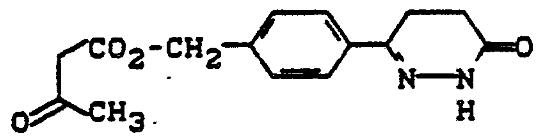

20 g 4-(6-Oxo-1,4,5,6-tetrahydropyridazin-3-yl)benzylalkohol (Beispiel 3) werden in 50 ml Tetrahydrofuran suspendiert und nach Zugabe von 100 mg 4-Dimethylaminopyridin unter Rückfluß mit 10,7 g Diketen umgesetzt. Man läßt 2 Stunden nachreagieren. Die Verbindung kristallisiert nach Abdampfen eines Teils des Tetrahydrofurans und Zugabe von Ethanol.
Ausbeute: 20,6 g (73% der Theorie)
Schmp.: >200°C

Beispiel 5

β-Aminocrotonsäure-[4-(6-oxo-1,4,5,6-tetrahydropyridazin-3-yl(benzyl]ester

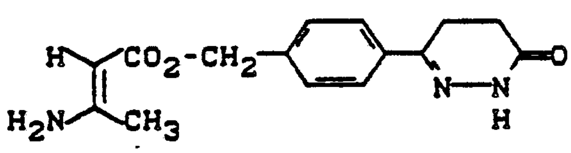

10 g Acetessigsäure-[4-(6-oxo-1,4,5,6-tetrahydropyridazin-3-yl)benzyl]ester (Beispiel 4) werden in 100 ml trockenem Tetrahydrofuran suspendiert und bei Rückflußtemperatur mit Ammoniakgas gesättigt. Man läßt über Nacht erkalten, dampft das Tetrahydrofuran ab und kocht den Rückstand mit Isopropanol aus.
Ausbeute: 9,5 g (94% der Theorie)
Schmp.: 183°C

Beispiel 6

β-Aminocrotonsäure-2-[4-(6-oxo-1,4,5,6-tetrahydropyridazin-3-yl)phenyl]ethylester

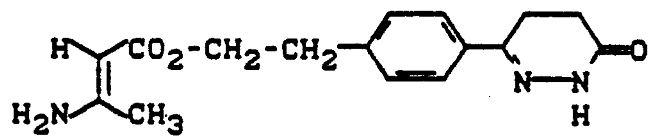

Aus analog Beispiel 3 hergestelltem 4-(6-Oxo-1,4,5,6-tetrahydropyridazin-3-yl)phenylethylalkohol und Diketen erhält man analog Beispiel 4 den Acetessigester, der nach Beispiel 5 mit Ammoniak in den gewünschten β-Aminocrotonsäureester überführt wird.
Ausbeute: 64% der Theorie

Beispiel 7

1,4-Dihydro-2,6-dimethyl-4-(3-nitrophenyl)-pyridin-3,5-dicarbonsäure-ethylester-[4-(6-oxo-1,4,5,6-tetrahydropyridazin-3-yl)benzyl]ester

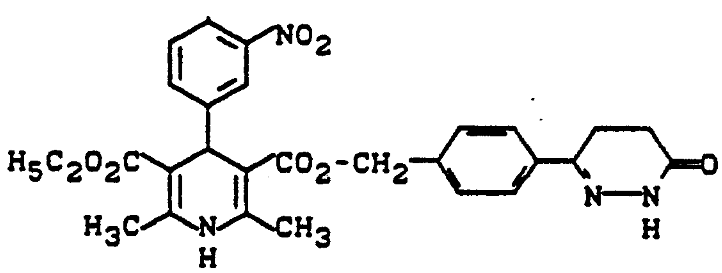

10,0 mmol 3-Nitrobenzaldehyd, 10 mmol Acetessigsäureethylester und 10 mmol β-Aminocrotonsäure-[4-(6-oxo-1,4,5,6-tetrahydropyridazin-3-yl)benzyl]ester werden in Isopropanol 12 h zum Rückfluß erhitzt. Das Produkt kristallisiert beim Abkühlen aus.
Schmp.: 258°C
Ausbeute: 62% der Theorie

Beispiel 8

2-Chlorbenzyliden-acetessigsäure-[4-(6-oxo-1,4,5,6-tetrahydropyridazin-3-yl)benzyl]ester

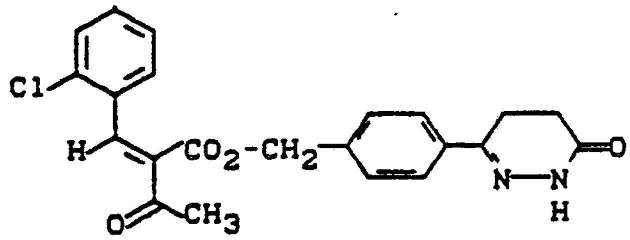

20 mmol 2-(n-Butyliminomethyl)-chlorbenzol und 20 mmol Acetessigsäure-[4-(6-oxo-1,4,5,6-tetrahydropyridazin-3-yl)benzyl]ester werden gleichzeitig in 30 ml Acetanhydrid gegeben und nach kurzem Erwärmen 24 h bei Raumtemperatur gerührt. Der Ansatz wird dann auf 250 ml Eiswasser gegeben, 2 h gerührt, der Niederschlag wird abfiltriert, mit wenig Ethanol ausgekocht.
Schmp.: 192°C
Ausbeute: 63% der Theorie

Beispiel 9

4-(2-Chlorphenyl)-1,4-dihydro-2,6-dimethyl-pyridin-3,5-dicarbonsäure-isopropylester-[4-(6-oxo-1,4,5,6-tetrahydropiperazin-3-yl)benzyl]ester

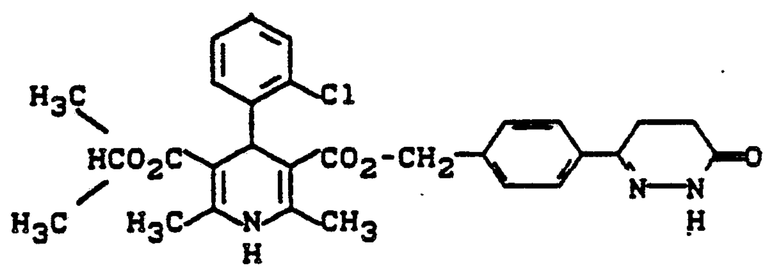

10 mmol β-Aminocrotonsäure-isopropylester und 2-Chlorbenzyliden-acetessigsäure-[4-(6-oxo-1,4,5,6-tetrahydropyridazin-3-yl)benzyl]ester werden in 30 ml Isopropanol 8 h zum Rückfluß erhitzt. Das Produkt kristallisiert nach dem Abkühlen beim Anreiben.
Schmp.: 211°C
Ausbeute: 73% der Theorie

Beispiel 10

1,4-Dihydro-2,6-dimethyl-4-(2-pyrid-2-yl-ethyl)pyridin-3,5-dicarbonsäure-mono-2-[4-(6-oxo-1,4,5,6-tetrahydropyridazin-3-yl)phenyl]ethylester

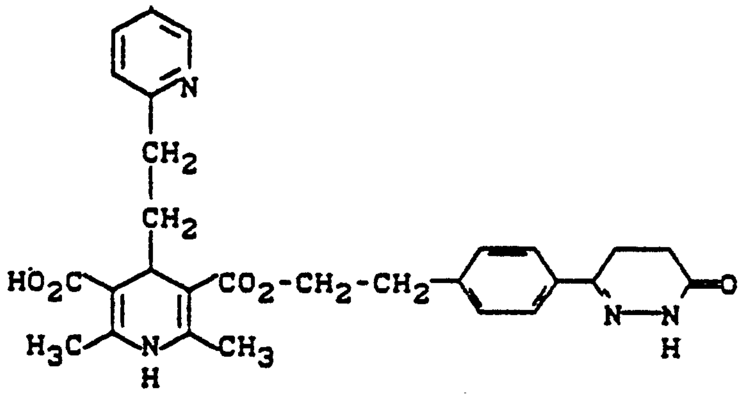

5 mmol 1,4-Dihydro-2,6-dimethyl-4-(2-pyrid-2-yl-ethyl)pyridin-3,5-dicarbonsäure-(2-cyanoethyl)-ester-2-[4-(6-oxo-1,4,5,6-tetrahydropyridazin-3-yl)phenyl]ethylester werden in 20 ml Dimethoxyethan gelöst und mit 15 mmol Natriumhydroxid in 30 ml Wasser versetzt und 6 h bei Raumtemperatur gerührt. Anschließend wird das Dimethoxyethan im Vakuum aus der Lösung abdestilliert, der wäßrige Rückstand wird mit Methylenchlorid extrahiert, anschließend auf pH 2 angesäuert und das ausgefallene Produkt nach Absaugen mit 10 ml Ethanol verrührt.
Schmp.: 218°C
Ausbeute: 82% der Theorie

Analog den beschriebenen Beispielen wurden die in der folgenden Tabelle aufgeführten erfindungsgemäßen Beispiele erhalten.

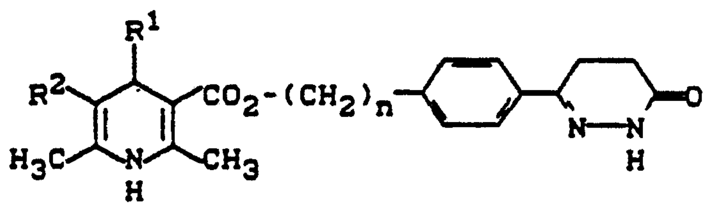

| Nr. | $R^1$ | $R^2$ | n | Schmp. [°C] |
|---|---|---|---|---|
| 11 | 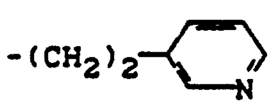 | 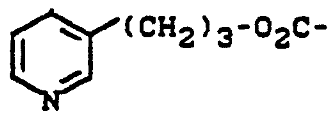 | 1 | Schaum |
| 12 | 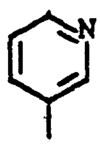 | 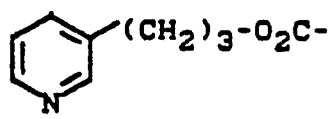 | 1 | 208 (Z) |
| 13 | 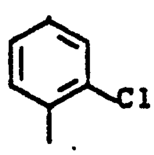 | $H_3CO_2C-$ | 1 | 259 |

| Nr. | $R^1$ | $R^2$ | n | Schmp. [°C] |
|---|---|---|---|---|
| 14 | 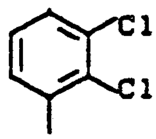 | $H_5C_2O_2C$- | 1 | 264 |
| 15 | 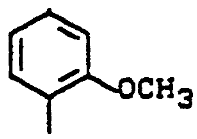 | $H_5C_2O_2C$- | 1 | 240 |
| 16 | $-(CH_2)_2$- 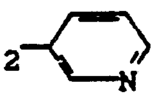 | $H_3CO_2C$- | 1 | 220 |
| 17 | $-(CH_2)_2$- 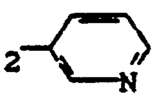 | $NC-CH_2-CH_2-O_2C$- | 1 | 182 |
| 18 | $-(CH_2)_2$- 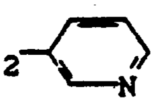 | $H_3C-CO$- | 1 | 220 |
| 19 | $-(CH_2)_2$- 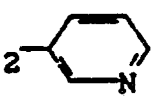 | NC- | 1 | 175 |
| 20 | $-(CH_2)_2$- 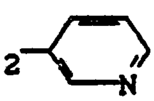 | $H_2N-CO$- | 1 | 128 |
| 21 | $-(CH_2)_2$- 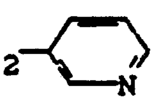 | $H_3CO_2C$- | 2 | 148 |
| 22 | $-(CH_2)_2$- 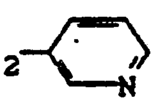 | 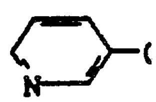 $-(CH_2)_3O_2C$- | 2 | 159 |

| Nr. | $R^1$ | $R^2$ | n | Schmp. [°C] |
|---|---|---|---|---|
| 23 | 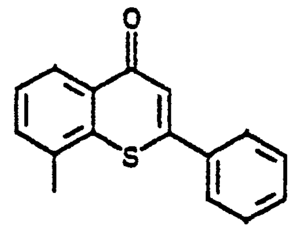 | 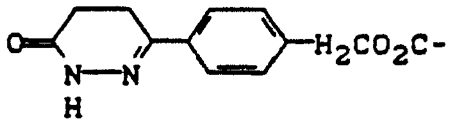 | 1 | 187 |
| 24 | 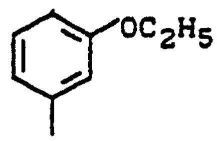 | 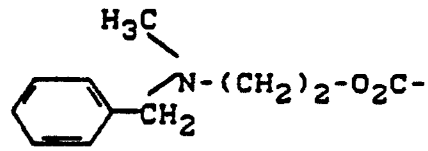 | 2 | 193 |
| 25 | 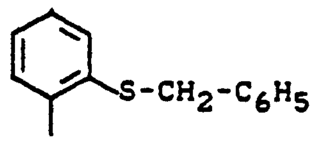 | 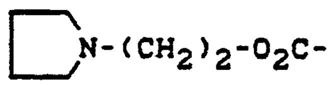 | 1 | 216 |
| 26 | 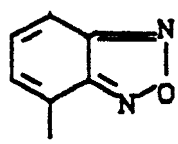 | $H_3CO-CH_2-CH_2-O_2C-$ | 1 | 227 |
| 27 | 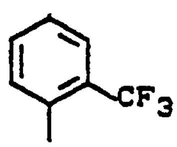 | $Br-(CH_2)_3-O_2C-$ | 2 | 163 |

**Ansprüche**

1. 1,4-Dihydropyridine der allgemeinen Formel (I)

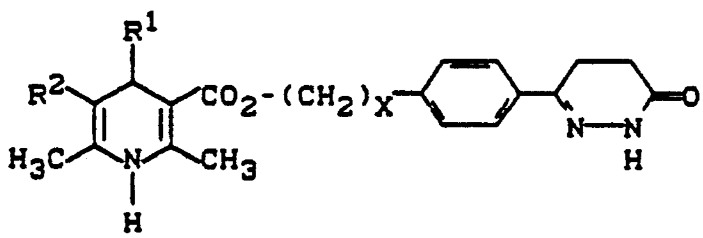 (I),

in welcher
$R^1$ - für $C_6$-$C_{14}$-Aryl steht, das bis zu vierfach gleich oder verschieden substituiert sein kann durch Halogen, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Trifluormethylthio oder durch gegebenenfalls durch Nitro, Cyano, Trifluormethyl, $C_1$-$C_4$-Alkyl oder $C_1$-$C_4$-

Alkoxy substituiertes Benzyl, Benzyloxy oder Benzylthio, oder

-für einen gegebenenfalls durch Halogen, Phenyl oder $C_1$-$C_4$-Alkyl substituierten Heterocyclus aus der Reihe Pyrryl, Thienyl, Furyl, Pyrazolyl, Imidazolyl, Oxazolyl, Thiazolyl, Pyridyl, Pyridazinyl, Pyrimidyl, Chinolyl, Benzoxadiazolyl, Chromenyl oder Thiochromenyl steht, oder

-für geradkettiges, verzweigtes oder cyclisches Alkyl mit bis zu 6 Kohlenstoffatomen steht, das gegebenenfalls substituiert ist durch Pyridyl, Pyrimidyl, Halogen oder Nitro,

$R^2$ - für Cyano oder für eine Gruppe der Formel

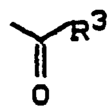

steht,

worin

$R^3$ - geradkettiges, verzweigtes oder cyclisches Alkyl mit bis zu 8 Kohlenstoffatomen bedeutet, oder

-eine Gruppe der Formel

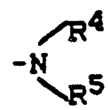

oder $-OR^6$ bedeutet,

worin

$R^4$, $R^5$ -gleich oder verschieden sind und

-für Wasserstoff, $C_1$-$C_6$-Alkyl, Phenyl oder Benzyl stehen,

und

$R^6$ -für Wasserstoff oder

-für geradkettiges, verzweigtes oder cyclisches Alkyl steht, das substituiert sein kann durch Halogen, Cyano, Hydroxy, $C_1$-$C_8$-Alkoxy, Pyridyl, Pyrimidyl, Thienyl, Furyl oder durch gegebenenfalls durch Halogen, Nitro, Cyano, Trifluormethyl, $C_1$-$C_4$-Alkyl, $C_1$-$C_4$-Alkoxy oder eine Gruppe der Formel

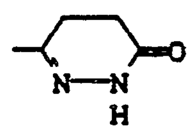

substituiertes Phenyl oder

durch Phenoxy, Phenylthio, Phenylsulfonyl oder

-durch eine Gruppe der Formel

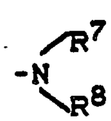 ,

worin

$R^7$, $R^8$ -gleich oder verschieden sind und

-für Wasserstoff, $C_1$-$C_6$-Alkyl, Phenyl, Benzoyl, Phenethyl, Acetyl, Benzoyl, Phenylsulfonyl oder $C_1$-$C_4$-Alkylsulfonyl stehen,

oder

$R^7$ und $R^8$ gemeinsam mit den Stickstoffatom einen 5-bis 7-gliedrigen Ring bilden, der gegebenenfalls als weiteres Heteroatom ein Sauerstoff, Schwefel, NH, N-Phenyl oder N-$C_1$-$C_4$-Alkyl oder N-Benzyl enthalten kann,

und
X - für eine Zahl von 0 bis 6 steht
und deren physiologisch unbedenkliche Salze.

2. Verbindungen der allgemeinen Formel (I) gemäß Anspruch 1, in welcher
$R^1$ - für Phenyl oder Naphthyl steht, das bis zu dreifach gleich oder verschieden substituiert sein kann durch Fluor, Chlor, Brom, Nitro, Cyano, $C_1$-$C_4$-Alkyl, $C_1$-$C_4$-Alkoxy, $C_1$-$C_4$-Alkylthio, Trifluormethyl, Trifluormethoxy, Difluormethoxy, Trifluormethylthio, durch gegebenenfalls durch Nitro, Fluor, Chlor, Brom, Trifluormethyl, Methyl oder Methoxy substituiertes Benzyloxy oder Benzylthio, oder

-für einen gegebenenfalls durch Methyl, Fluor, Chlor, Brom oder Phenyl substituierten Heterocyclus aus der Reihe Thienyl, Furyl, Pyridyl, Pyrimidyl, Chinolyl, Benzoxadiazolyl oder Thiochromenyl steht, oder

-für geradkettiges oder verzweigtes, gegebenenfalls durch Fluor, Chlor, Brom oder Pyridyl substituiertes Alkyl mit bis zu 4 Kohlenstoffatomen steht,
$R^2$ - für Cyano oder für eine Gruppe der Formel

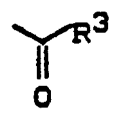

steht,
worin
$R^3$ - für geradkettiges, verzweigtes oder cyclisches Alkyl mit bis zu 6 Kohlenstoffatomen steht, oder
-für eine Gruppe der Formel

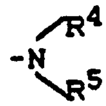

oder $-OR^6$ steht,
wobei
$R^4$, $R^5$ -gleich oder verschieden sind und
-für Wasserstoff, $C_1$-$C_4$-Alkyl oder Phenyl stehen,
und
$R^6$ -für Wasserstoff oder

-für geradkettiges oder verzweigtes Alkyl mit bis zu 8 Kohlenstoffatomen steht, das substituiert sein kann durch bis zu 7 Fluor, durch Chlor, Brom, Cyano, Hydroxy, $C_1$-$C_6$-Alkoxy, Pyridyl, Phenoxy oder durch gegebenenfalls durch Fluor, Chlor, Brom, Nitro, Cyano, Trifluormethyl, $C_1$-$C_2$-Alkyl, $C_1$-$C_2$-Alkoxy oder eine Gruppe der Formel

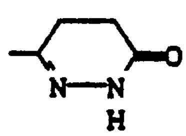

substituertes Phenyl, oder
durch eine Gruppe der Formel

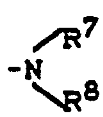,

wobei
$R^7$, $R^8$ -gleich oder verschieden sind und

-für Wasserstoff, $C_1$-$C_4$-Alkyl, Phenyl, Benzyl oder Acetyl stehen,
oder
$R^7$ und $R^8$ gemeinsam einen 5-bis 7-gliedrigen Ring bilden, der gegebenenfalls als weiteres Heteroatom Schwefel oder Sauerstoff enthält,
und
X - für eine Zahl von 0 bis 5 steht
und deren physiologisch unbedenkliche Salze.

3. Verbindungen der allgemeinen Formel (I) gemäß Anspruch 1, in welcher
$R^1$ - für Phenyl steht, das bis zu zweifach gleich oder verschieden substituiert sein kann durch Fluor, Chlor, Nitro, Cyano, $C_1$-$C_4$-Alkyl, $C_1$-$C_4$-Alkoxy, Trifluormethyl, Benzyloxy oder Benzylthio, oder
-für Thienyl, Furyl, Pyridyl, Benzoxadiazolyl oder
-für einen Heterocyclus der Formel

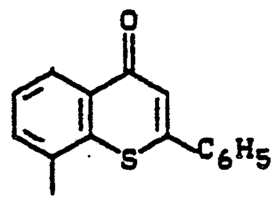

steht, oder
-für gegebenenfalls durch α-, β-oder γ-Pyridyl substituiertes Methyl oder Ethyl steht,
und
$R^2$ - für Cyano oder eine Gruppe der Formel

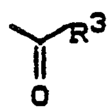

steht,
worin
$R^3$ - für geradkettiges oder verzweigtes Alkyl mit bis zu 4 Kohlenstoffatomen steht,
oder
-für einen Rest der Formel

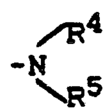

oder $-OR^6$ steht,
wobei
$R^4$, $R^5$ -gleich oder verschieden sind und
-für Wasserstoff, Methyl oder Ethyl stehen
und
$R^6$ -für Wasserstoff oder
-für geradkettiges oder verzweigtes Alkyl mit bis zu 6 Kohlenstoffatomen steht, das durch bis zu 3 Fluor, Chlor, Brom, Cyano, Hydroxy, $C_1$-$C_4$-Alkoxy, α-, β-oder γ-Pyridyl oder durch eine Gruppe der Formel

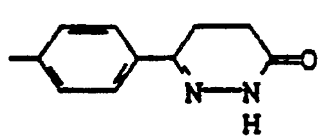

oder

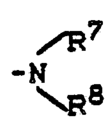

substituiert sein kann,
worin
$R^7$, $R^8$ -gleich oder verschieden sind und
-für Wasserstoff, Methyl, Ethyl oder Benzyl stehen,
oder
$R^7$ und $R^8$ gemeinsam einen Pyrrolidino-oder Piperidinoring bilden,
und
X - für eine Zahl 1 bis 4 steht
und deren physiologisch unbedenkliche Salze.

4. Verfahren zur Herstellung von 1,4-Dihydropyridinen der allgemeinen Formel (I)

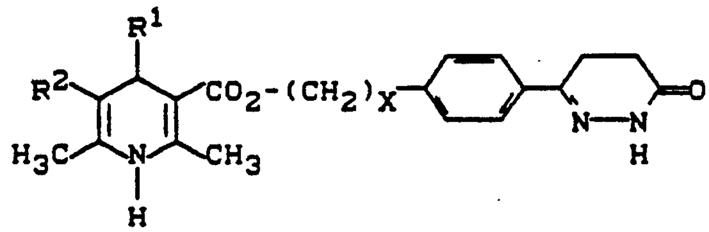
(I),

in welcher
$R^1$ - für $C_6$-$C_{14}$-Aryl steht, das bis zu vierfach gleich oder verschieden substituiert sein kann durch Halogen, Nitro, Cyano, $C_1$-$C_6$-Alkyl, $C_1$-$C_6$-Alkoxy, $C_1$-$C_6$-Alkylthio, Trifluormethyl, Trifluormethoxy, Difluormethoxy, Trifluormethylthio oder durch gegebenenfalls durch Nitro, Cyano, Trifluormethyl, $C_1$-$C_4$-Alkyl oder $C_1$-$C_4$-Alkoxy substituiertes Benzyl, Benzyloxy oder Benzylthio, oder
-für einen gegebenenfalls durch Halogen, Phenyl oder $C_1$-$C_4$-Alkyl substituierten Heterocyclus aus der Reihe Pyrryl, Thienyl, Furyl, Pyrazolyl, Imidazolyl, Oxazolyl, Thiazolyl, Pyridyl, Pyridazinyl, Pyrimidyl, Chinolyl, Benzoxadiazolyl, Chromenyl oder Thiochromenyl steht, oder
-für geradkettiges, verzweigtes oder cyclisches Alkyl mit bis zu 6 Kohlenstoffatomen steht, das gegebenenfalls substituiert ist durch Pyridyl, Pyrimidyl, Halogen oder Nitro,
$R^2$ - für Cyano oder für eine Gruppe der Formel

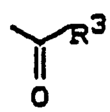

steht,
worin
$R^3$ - geradkettiges, verzweigtes oder cyclisches Alkyl mit bis zu 8 Kohlenstoffatomen bedeutet, oder
-eine Gruppe der Formel

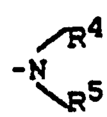

oder $-OR^6$ bedeutet,
worin
$R^4$, $R^5$ -gleich oder verschieden sind und
-für Wasserstoff, $C_1$-$C_6$-Alkyl, Phenyl oder Benzyl stehen,
und
$R^6$ -für Wasserstoff oder
-für geradkettiges, verzweigtes oder cyclisches Alkyl steht, das substituiert sein kann durch Halogen, Cyano, Hydroxy, $C_1$-$C_8$-Alkoxy, Pyridyl, Pyrimidyl, Thienyl, Furyl oder durch gegebenenfalls durch Halogen, Nitro, Cyano, Trifluormethyl, $C_1$-$C_4$-Alkyl, $C_1$-$C_4$-Alkoxy oder eine Gruppe der Formel

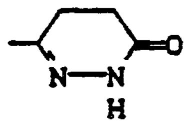

substituiertes Phenyl oder
durch Phenoxy, Phenylthio, Phenylsulfonyl oder
-durch eine Gruppe der Formel

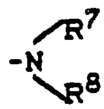

worin
$R^7$, $R^8$ -gleich oder verschieden sind und
-für Wasserstoff, $C_1$-$C_6$-Alkyl, Phenyl, Benzoyl, Phenethyl, Acetyl, Benzoyl, Phenylsulfonyl oder $C_1$-$C_4$-Alkylsulfonyl stehen,
oder
$R^7$ und $R^8$ gemeinsam mit den Stickstoffatom einen 5-bis 7-gliedrigen Ring bilden, der gegebenenfalls als weiteres Heteroatom ein Sauerstoff, Schwefel, NH, N-Phenyl oder N-$C_1$-$C_4$-Alkyl oder N-Benzyl enthalten kann,
und
X - für eine Zahl von 0 bis 6 steht
und deren physiologisch unbedenkliche Salze, dadurch gekennzeichnet, daß man
[A] Aldehyde der allgemeinen Formel (II)

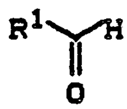
(II)

in welcher
$R^1$ die angegebene Bedeutung hat,
mit Ketonen der allgemeinen Formel (III),

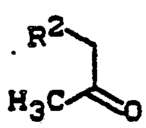
(III)

in welcher
$R^2$ die angegebene Bedeutung hat,
und Enaminen der allgemeinen Formel (IV)

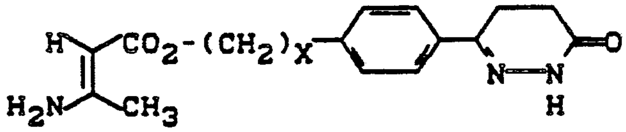
(IV)

in welcher
X die angegebene Bedeutung hat,
in Gegenwart inerter Lösemittel umsetzt,
oder indem man

[B] Aldehyde der allgemeinen Formel (II), mit Ketonen der allgemeinen Formel (V),

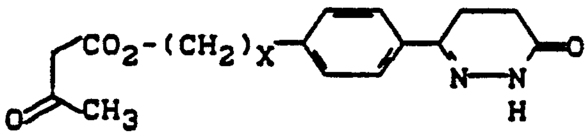 (V)

in welcher X die angegebene Bedeutung hat, und Enaminen der allgemeinen Formel (VI)

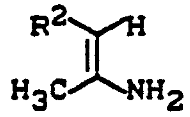 (VI),

in welcher $R^2$ die angegebene Bedeutung hat, in Gegenwart inerter Lösemittel umsetzt, oder indem man

[C] Ketone der allgemeinen Formel (III) mit Ammoniak und Ylidenverbindungen der allgemeinen Formel (VII)

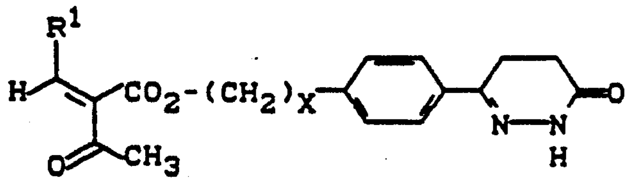 (VII)

in welcher $R^1$ und X die angegebene Bedeutung haben, in Gegenwart inerter Lösemittel umsetzt, oder indem man

[D] Ketone der allgemeinen Formel (V), mit Ammoniak und Ylidenverbindungen der allgemeinen Formel (VIII)

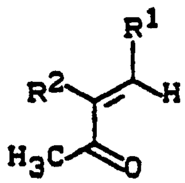 (VIII)

in welcher $R^1$ und $R^2$ die angegebene Bedeutung haben, in Gegenwart inerter Lösemittel umsetzt, oder indem man

[E] Enamine der allgemeinen Formel (VI) mit Ylidenverbindungen der allgemeinen Formel (VII) in Gegenwart inerter Lösemittel umsetzt, oder indem man

[F] Enamine der allgemeinen Formel (IV) mit Ylidenverbindungen der allgemeinen Formel (VII) in Gegenwart inerter Lösemittel umsetzt.

5. Verfahren gemäß Anspruch 4, dadurch gekennzeichnet, daß man die Umsetzung in Gegenwart von inerten Lösungsmitteln bei Temperaturen zwischen 10 und 200°C durchführt.

6. Arzneimittel enthaltend mindestens eine Verbindung der allgemeinen Formel (I) gemäß Anspruch 1.

7. Verfahren zur Herstellung von Arzneimitteln, dadurch gekennzeichnet, daß man Verbindungen der allgemeinen Formel (I) gemäß Anspruch 1 gegebenenfalls unter Verwendung von Hilfs-und Trägerstoffen in eine geeignete Applikationsform überführt.

8. Verbindungen der allgemeinen Formel (I) gemäß Anspruch 1 zur Verwendung bei der Bekämpfung von Erkrankungen.

9. Verwendung von Verbindungen der allgemeinen Formel (I) gemäß Anspruch 1 bei der Herstellung von kreislaufwirksamen Arzneimitteln.

10. Verwendung von Verbindungen der allgemeinen Formel (I) gemäß Anspruch 1 bei der Bekämpfung von Kreislauferkrankungen.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 88105148.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 445 852 (BAYER)<br>* Formel I; Seite 24, Zeilen 1-5 *<br>-- | 1,9 | C 07 D 401/12<br>C 07 D 401/14<br>C 07 D 409/14<br>C 07 D 413/14<br>A 61 K 31/505 |
| A | DE - A1 - 3 431 862 (BAYER)<br>* Formel I; Beispiel 8 *<br>---- | 1 | |

RECHERCHIERTE SACHGEBIETE (Int. Cl.4)

C 07 D 401/00
C 07 D 409/00
C 07 D 413/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9
Unvollständig recherchierte Patentansprüche: -
Nicht recherchierte Patentansprüche: 10
Grund für die Beschränkung der Recherche:

(Art. 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 06-06-1988 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03 82